# EUROPEAN PATENT SPECIFICATION

(11) **EP 2 190 493 B1**
(45) Date of publication and mention of the grant of the patent: **27.06.2012**
(21) Application number: 08833080.8
(22) Date of filing: 24.09.2008
(51) Int. Cl.: A61L 31/02, A61L 31/14, A61L 31/16

(54) **MEDICAL DEVICES HAVING A METAL PARTICULATE COMPOSITION FOR CONTROLLED DIFFUSION**
MEDIZINISCHE VORRICHTUNGEN MIT METALLPARTIKELZUSAMMENSETZUNG FÜR GESTEUERTE DIFFUSION
DISPOSITIFS MÉDICAUX AYANT UNE COMPOSITION PARTICULAIRE MÉTALLIQUE POUR DIFFUSION CONTRÔLÉE

(30) Priority: 24.09.2007 US 860233
(43) Date of publication of application: 02.06.2010
(73) Proprietor: Boston Scientific Scimed, Inc., Maple Grove, MN 55311-1566 (US)
(72) Inventor: CLARKE, John, Galway (IE); MCMORROW, David, Galway (IE); FLANAGAN, Aiden, Galway (IE); O'CONNOR, Tim, Galway (IE); WEBER, Jan, 6228 GJ Maastricht (NL); O'BRIEN, Barry, J., Galway (IE)
(74) Representative: Peterreins, Frank
(86) International application number: PCT/US2008/077496
(87) International publication number: WO 2009/042667

(56) References cited:
- EP-A1- 1 466 634
- EP-A2- 0 875 218
- US-A1- 2004 039 438
- US-A1- 2005 149 170
- US-A1- 2006 045 901

## Description

### TECHNICAL FIELD

This invention relates to medical devices, and more particularly, to medical devices that utilize metallic particles to control the release of one or more therapeutic agents.

### BACKGROUND OF THE INVENTION

The in-situ delivery of therapeutic agents within the body of a patient is common in the practice of modern medicine. In-situ delivery of therapeutic agents is often implemented using medical devices that may be temporarily or permanently placed at a target site within the body. These medical devices can be maintained, as required, at their target sites for short or prolonged periods of time in order to deliver therapeutic agents to the target site.
US2004/0039438 discloses a stent comprising a base layer to which is bonded metallic particles in order to leave interstices for retaining and dispensing drugs.
EP0875218 discloses a metallic stent having a plurality of pores in the metal which are loaded with medication.
EP1466634 discloses a drug-eluting stent for controlled delivery comprising a plurality of reservoirs, a drug polymer positioned in the reservoirs and a polymer layer.
US2006/0045901 discloses a stent, a drug eluting surface coated with a coating of particles made of polymers.

In some cases however, delivery of the biologically active material to the body tissue immediately after insertion or implantation of the medical device may not be needed or desired. For instance, if a stent is used to prevent the occurrence of restenosis after balloon angioplasty, it may be more desirable to wait until restenosis occurs or begins to occur in a body lumen that has been stented with a drug-coated stent before the drug is released. Therefore, there is a need for insertable or implantable medical devices that can provide delayed and/or controlled delivery of biologically active materials when such materials are required by the patient after implantation of the medical device.

Current techniques for the in-situ delivery of therapeutic agents in a controlled manner often involve the use of a polymer coating on the insertable or implanatable medical device to contain the agents and control its release rate. The polymer coating, however, can sometimes cause an inflammatory response in the tissue with which it comes in contact. For instance, when a Drug Eluting Stent (DES) is implanted in a vessel, the inflammatory response can cause a reduction in the diameter of the vessel lumen within the stent. The inflammatory response can lead to late in stent thrombosis.

### SUMMARY OF THE INVENTION

In accordance with the present invention, an implantable or insertable medical device is provided which includes as components: (a) a substrate component comprising one or more depressions that is at least partially filled with a therapeutic agent-containing material that comprises a first therapeutic agent, and (b) a metallic particulate composition disposed in the depression such that it regulates transport of chemical species between the depression and the exterior of the device upon implantation or insertion of the device into a subject.

In accordance with one aspect of the invention, the particulate composition and the substrate may be magnetic such that the particulate composition is retained in the cavity by magnetic force the particulate composition comprises compacted particles.

In accordance with the invention, the particulate composition comprises metallic particles.

In accordance with one aspect of the invention, the metallic particles may be biodisintegratable.

In accordance with one aspect of the invention, the particulate composition may include porous channels through which the first therapeutic agent can be released.

In accordance with one aspect of the invention, a seal may be disposed over the particulate composition in the depression to delay release of the therapeutic agent.

In accordance with one aspect of the invention, the substrate component may comprise a plurality of depressions.

In accordance with one aspect of the invention, the depression may be a blind hole or a trench.

In accordance with one aspect of the invention, the medical device may be adapted for implantation or insertion into the coronary vasculature, peripheral vascular system, esophagus, trachea, colon, biliary tract, urogenital system, or brain.

In accordance with one aspect of the invention, the medical device may be selected from a drug delivery device, an implant, a stent, a graft, a filter, a catheter, a defibrillator, a chronic rhythm management lead and a neuromodulation device.

In accordance with one aspect of the invention, the therapeutic-agent-containing material may further comprise a material in addition to said first therapeutic agent

In accordance with one aspect of the invention, the therapeutic-agent-containing material may further comprise a second therapeutic agent.

These and other embodiments and advantages of the present invention will become readily apparent to those of ordinary skill in the art upon review of the Detailed Description and Claims to follow.

### BRIEF DESCRIPTION OF THE DRAWINGS

Figs. 1A-1B are schematic cross-sectional views illustrating a substrate from which a tubular medical device may be formed in accordance with an embodiment of the invention.

Fig. 2A is a schematic perspective view of a stent in accordance with an embodiment of the invention. Fig. 2B is a schematic cross-sectional view taken along line b--b of Fig. 2A. Fig. 2C is a schematic perspective view of a portion of the stent of Fig. 2A.

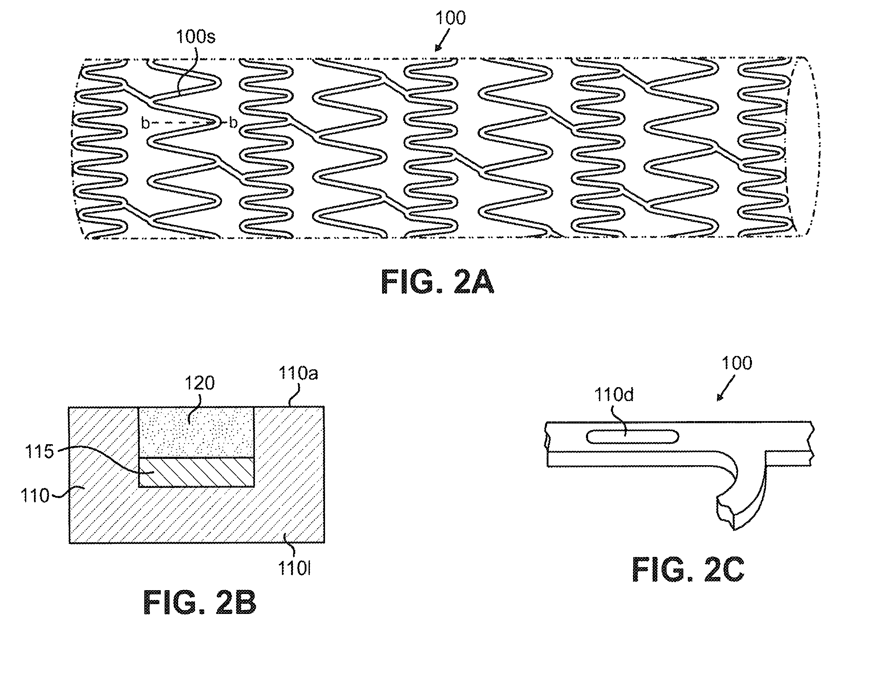

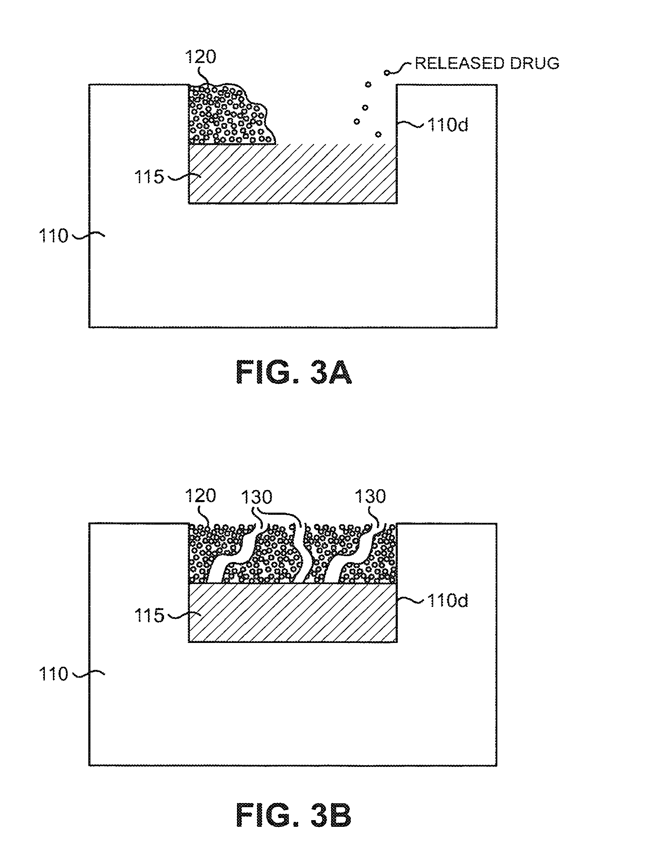

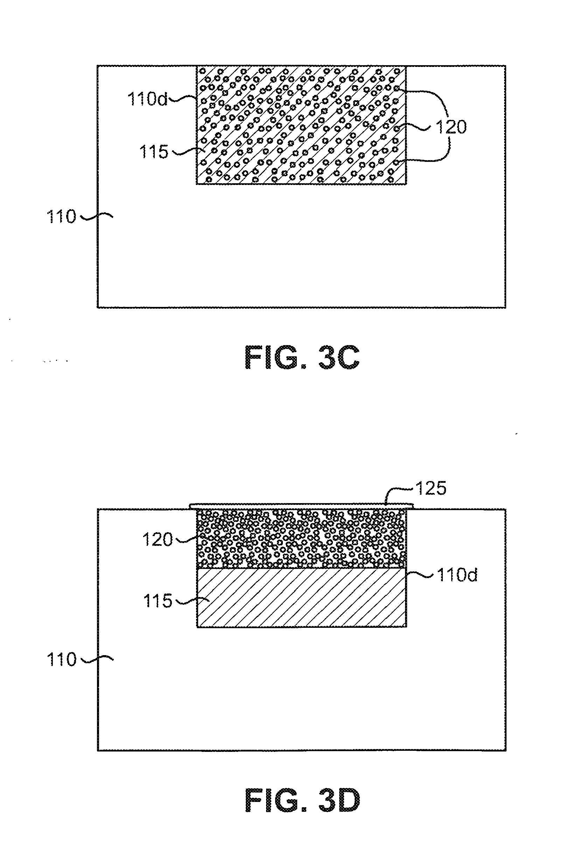

Fig. 3A is a schematic cross-sectional view of the invention taken along line b--b of Fig. 2A after a portion of the metallic particulate composition has disintegrated. Fig. 3B is a schematic cross-sectional view of one alternative embodiment of the invention taken along line b--b of Fig. 2A in which porous channels are provided through the metallic particulate composition. Fig. 3C is a schematic cross-sectional view of another alternative embodiment of the invention taken along line b--b of Fig. 2A in which the particles in the metallic particulate composition are intermixed with the therapeutic agent-containing composition. Fig. 3D is a schematic cross-sectional view of another alternative embodiment of the invention taken along line b--b of Fig. 2A in which a seal is provided over the depression.

Figs. 4A-4G and 5A-5E are schematic top views illustrating various depression configurations and arrays of the same, which may be employed in various embodiments of the invention.

Figs. 6A-6E are schematic cross-sectional views illustrating various depression configurations, which may be employed in various embodiments of the invention.

Figs. 7-9 are various alternative embodiments of a tubular medical device that may be formed in accordance with the invention.

### DETAILED DESCRIPTION

According to an aspect of the present invention, implantable or insertable medical devices are provided which contain the following: (a) a substrate having one or more depressions that contain at least one therapeutic agent and (b) a collection of metallic particles in one or more of the depressions and which cover the therapeutic agent. The collection of metallic particles regulate transport of chemical species (e.g., in many embodiments, the therapeutic agent, among others) between the therapeutic-agent-containing depressions and the exterior of the device. The metallic particles may be biodisintegrable particles (i.e., materials, that, upon placement in the body, are dissolved, degraded, eroded, resorbed, and/or otherwise removed from the placement site over the anticipated placement period). As the metallic particles disintegrate, the rate of transport of the chemical species between the depressions and the exterior of the device increases in a manner that can be controlled by choosing the type, size packing and layer thickness of metallic particle layer. Transport of the chemical species may also be regulated by controlling the degree of porosity of the chemical species through the collection of metallic particles. The use of metallic particles can advantageously avoid the use of a polymer coating to control the release of the chemical species.

Implantable or insertable medical devices which can be constructed in accordance with the invention vary widely and include, for example, stents (including coronary vascular stents, peripheral vascular stents, cerebral, urethral, ureteral, biliary, tracheal, gastrointestinal and esophageal stents), stent coverings, stent grafts, vascular grafts, abdominal aortic aneurysm (AAA) devices (e.g., AAA stents, AAA grafts), vascular access ports, dialysis ports, catheters (*e.g*., urological catheters or vascular catheters such as balloon catheters and various central venous catheters), guide wires, filters (*e.g*., vena cava filters and mesh filters for distil protection devices), embolization devices including cerebral aneurysm filler coils (including Guglilmi detachable coils and metal coils), septal defect closure devices, drug depots that are adapted for placement in an artery for treatment of the portion of the artery distal to the device, myocardial plugs, pacemakers, leads including pacemaker leads, defibrillation leads, and coils, ventricular assist devices including left ventricular assist hearts and pumps, total artificial hearts, shunts, valves including heart valves and vascular valves, anastomosis clips and rings, cochlear implants, tissue bulking devices, and tissue engineering scaffolds for cartilage, bone, skin and other in vivo tissue regeneration, sutures, suture anchors, tissue staples and ligating clips at surgical sites, cannulae, metal wire ligatures, urethral slings, hernia "meshes", artificial ligaments, orthopedic prosthesis such as bone grafts, bone plates, fins and fusion devices, joint prostheses, orthopedic fixation devices such as interference screws in the ankle, knee, and hand areas, tacks for ligament attachment and meniscal repair, rods and pins for fracture fixation, screws and plates for craniomaxillofacial repair, dental implants, or other devices that are implanted or inserted into the body.

The medical devices of the present invention include, for example, implantable and insertable medical devices that are used for systemic diagnosis or treatment, as well as those that are used for the localized diagnosis or treatment of any mammalian tissue or organ. Non-limiting examples are tumors; organs including the heart, coronary and peripheral vascular system (referred to overall as "the vasculature"), the urogenital system, including kidneys, bladder, urethra, ureters, prostate, vagina, uterus and ovaries, eyes, ears, spine, nervous system, lungs, trachea, esophagus, intestines, stomach, brain, liver and pancreas, skeletal muscle, smooth muscle, breast, dermal tissue, cartilage, tooth and bone.

Medical devices benefiting from the present invention thus include a variety of implantable and insertable medical devices including devices for insertion into and/or through a wide range of body lumens, for purposes of diagnosis or treatment, several of which are recited above, including lumens of the cardiovascular system such as the heart, arteries (e.g., coronary, femoral, aorta, iliac, carotid and vertebro-basilar arteries) and veins, lumens of the genitourinary system such as the urethra (including prostatic urethra), bladder, ureters, vagina, uterus, spermatic and fallopian tubes, the nasolacrimal duct, the eustachian tube, lumens of the respiratory tract such as the trachea, bronchi, nasal passages and sinuses, lumens of the gastrointestinal tract such as the esophagus, gut, duodenum, small intestine, large intestine, rectum, biliary and pancreatic duct systems, lumens of the lymphatic system, the major body cavities (peritoneal, pleural, pericardial) and so forth.

As used herein, terms such as "treatment" and "therapy" refers to the prevention of a disease or condition, the reduction or elimination of symptoms associated with a disease or condition, or the substantial or complete elimination of a disease or condition.

Preferred subjects for treatment or diagnosis are vertebrate subjects, for example, humans, livestock and pets.

In some embodiments, the substrate from which the medical device is formed has a tubular configuration (e.g., stents, tubing, etc.). In such embodiments, the one or more depressions may be provided within the abluminal surface of the tubular substrate. Alternatively, the one or more depressions may be provided within the luminal surface of the tubular substrate. As another alternative, among others, the one or more depressions may be provided within each of the luminal and abluminal surfaces of the tubular substrate.

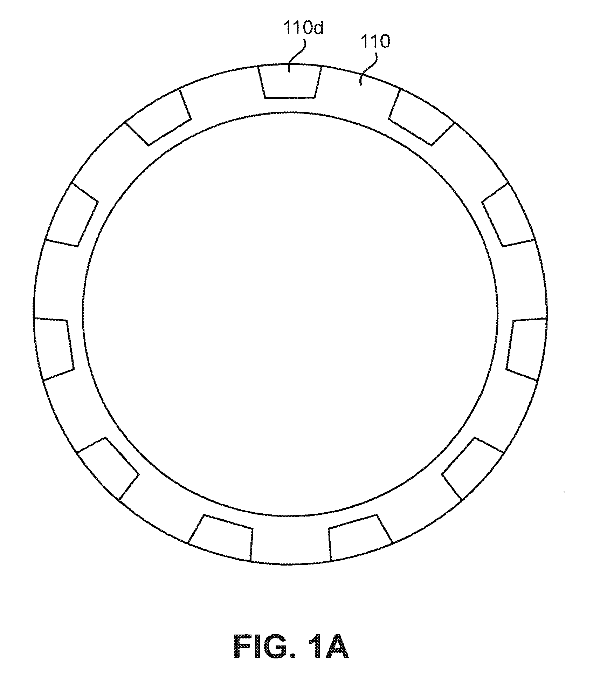

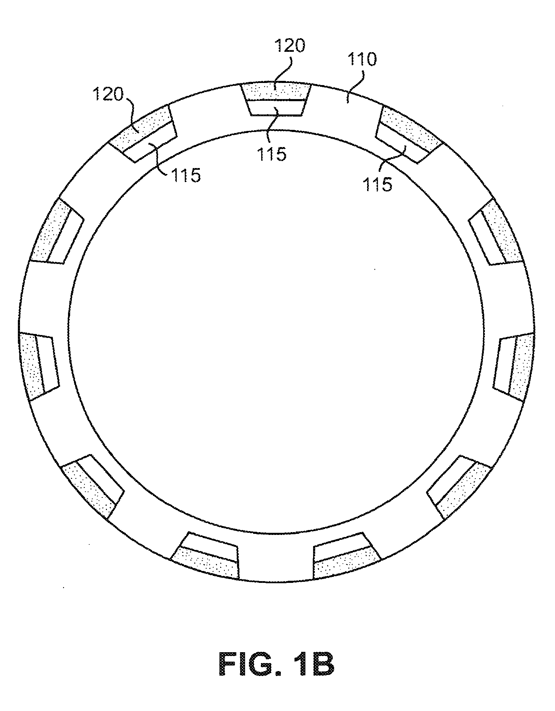

By way of example, Fig. 1A is a schematic cross-section illustrating a tubular medical device substrate 110, which contains depressions 110d on its outer (abluminal) surface, which can be filled with a therapeutic-agent-containing composition 115 as shown in Fig. 1B. The depressions can be loaded with the composition 115 using, for instance, solvent carriers and evaporation techniques. Alternatively, the composition 115 can be loaded as crystalline or amorphous powder. The therapeutic-agent-containing composition 115 may consist essentially of one or more therapeutic agents, or it may contain further optional agents such as polymer matrix materials, diluents, excipients or fillers. Moreover, all of the depressions 110d may be filled with the same therapeutic-agent-containing composition 115, or some depressions may be filled with a first therapeutic-agent-containing composition while other depressions may be filled with a different therapeutic-agent-containing composition, among other possibilities. A metallic particulate composition 120 covers the therapeutic-agent-containing composition 115 and in some cases may fill the remainder of each of the depressions 110d.

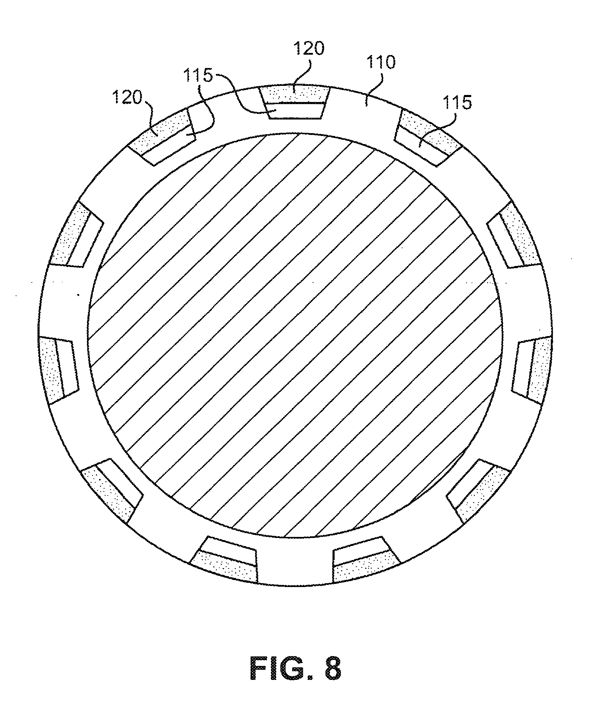

A schematic cross-sectional illustration of another substrate that may be employed is shown in Fig. 8. This substrate is similar to that of Figs. 1A and 1B, except that the substrate 110 is of circular (solid) cross-section rather than annular (hollow) cross-section. Examples of medical devices that can be formed from this type of substrate include, for example, embolic spheres, embolic rods, and orthopedic implants, among many others.

One example of medical device that may be formed from the tubular substrate shown in Figs. 1A and 1B is a stent. FIG. 2A shows a schematic perspective view of an illustrative stent 100 which contains a number of interconnected struts 100s. Fig. 2B is a cross-section taken along line b--b of strut 100s of stent 100 of Fig. 2A, which has an abluminal surface 100a and a luminal surface 1101. The following are shown in Fig. 2B: a strut substrate 110, a depression 110d, which is filled with a therapeutic-agent-containing composition 115, and a metallic particulate composition 120 that is disposed over the therapeutic-agent-containing composition 115. Fig. 2C is a perspective view of a portion of the stent 100 in Fig. 2A (designated by reference letter c) to shown the shape of the depression in the substrate 110.

Turning to Fig. 3A, which is a cross-section taken along line b--b of strut 100s of stent 100 similar to Fig. 2B after a portion of the metallic particulate composition 120 has biodisintegrated. The disintegration of the metallic particulate composition 120 allows a certain amount of the therapeutic-agent-containing composition 115 to be released. As the metallic particulate composition 120 continues to disintegrate, the amount of the therapeutic-agent-containing composition 115 that is released will increase, provided that sufficient quantities of the composition 115 remain available. The rate of disintegration, and therefore the rate and rate profile (i.e., the rate over time) at which the therapeutic-agent-containing composition 115 is released, can be controlled by varying a number of parameters, including, for example, the composition, degree of compaction, size, shape and surface area of the metallic particles forming the composition 120.

Examples of metallic materials from which the metallic particulate composition 120 may be selected include one or more of the following: biostable and biodisintegrable substantially pure metals, including gold, niobium, platinum, palladium, iridium, osmium, rhodium, titanium, zirconium, tantalum, tungsten, niobium, ruthenium, magnesium, zinc and iron, among others, and biostable and biodisintegrable metal alloys, including metal alloys comprising iron and chromium (e.g., stainless steels, including platinum-enriched radiopaque stainless steel), niobium alloys, titanium alloys, nickel alloys including alloys comprising nickel and titanium (e.g., Nitinol), alloys comprising cobalt and chromium, including alloys that comprise cobalt, chromium and iron (e.g., elgiloy alloys), alloys comprising nickel, cobalt and chromium (e.g., MP 35N), alloys comprising cobalt, chromium, tungsten and nickel (e.g., L605), and alloys comprising nickel and chromium (e.g., inconel alloys), and biodisintegrable alloys including alloys of magnesium, zinc and/or iron (and their alloys with combinations of each other an Ce, Ca, Zr and Li), among others. Further examples, not necessarily exclusive of the foregoing, include the biodegradable metallic materials described in U.S. Patent App. Pub. No. 2002/0004060 A1, entitled "Metallic implant which is degradable in vivo." These include substantially pure metals and metal alloys whose main constituent is selected from alkali metals, alkaline earth metals, iron, and zinc, for example, metals and metal alloys containing magnesium, iron or zinc as a main constituent and one or more additional constituents selected from the following: alkali metals such as Li, alkaline-earth metals such as Ca and Mg, transition metals such as Mn, Co, Ni, Cr, Cu, Cd, Zr, Ag, Au, Pd, Pt, Re, Fe and Zn, Group IIIa metals such as Al, and Group IVa elements such as C, Si, Sn and Pb.

The average size of the particles in particulate composition 120, in terms of volume, is typically within the range from 4 cubic nms to 1 cubic micrometer. However, the average particle size may be any other suitable range such as from 1 micron to 5 microns or 100 nanometers to 10 microns. The sizes should be determined based on various factors including a thickness of the layer of particles in the depression 110d and the desired release rate of the therapeutic agent-containing composition. Suitable particles are not limited to any particular shape.

The metallic particles in the composition 120 may be compacted in the depressions 110d in any suitable manner. Mechanical techniques to achieve such compaction into micron-sized depressions include micro-punching and sandblasting, for example. In micropunching, a punch having a suitable size to fit into the depressions 110d is used. A suitable quantity of the therapeutic-agent-containing composition and/or the metallic particles is positioned at the leading end of the punch and the punch is positioned in the depression. A suitable force is applied to the punch to provide the required level of compaction. As previously mentioned, the degree of compaction is one factor that will affect the rate at which the metallic particles disintegrate. In addition, the degree of compaction can affect the porosity of the metallic particle composition 120, which in turn can be used to further control the rate at which the therapeutic-agent-containing composition 115 is released.

If sandblasting is employed, a quantity of the therapeutic-agent-containing composition and/or the metallic particles is carried toward the substrate 110 using either a gas or a liquid stream. The particles are transported at a sufficient velocity so that they enter the depressions with sufficient kinetic energy to cause compaction to occur. If required, a secondary process such micropunching may be performed to further increase the degree of compaction. Suitable post-processing may also be used to remove excess particles or therapeutic-agent-containing composition

In some cases it may be desirable to perform the compaction process in two stages. For instance, if micron-sized depressions are used with nanometer-sized metallic particles, the particles may first be compacted and sintered prior to insertion in the depression. The compaction may be performed using ultrasonic energy (as is sometimes used to compact ceramic powder) followed by selective laser sintering, for example.

Fig. 3B shows a cross-section through a depression 110d similar to Fig. 3A except that the molecules of the therapeutic agent are released through porous channels 130 in the metallic particulate composition 120. The degree of compaction and the size and shape of the metallic particles 120 may be varied to control the pore sizes. Pore sizes may range, for example, from nanopores (i.e., pores having widths of 50 nm or less), which include micropores (i.e., pores having widths smaller than 2 nm) and mesopores (i.e., pores having a widths ranging from 2 to 50 nm), to macropores (i.e., pores having widths that are larger than 50 nm). In some cases the metallic composition may be configured to provide a nanoporous surface, which is one that comprises nanopores (commonly at least 10⁶, 10⁹, 10¹² or more nanopores per cm²), a microporous surface, which is one that comprises micropores, a mesoporous surface, which is one that comprises mesopores, or a macroporous surface, which is one that comprises macropores

In those embodiments in which porous channels 130 are employed, the metallic particle composition 120 may be disintegratable or even biostable. If biostable, the particle composition 120 remains an integral part of the medical device after the drug has been released. In addition, the therapeutic agent release rate or rate profile (i.e., the rate over time) will largely be controlled by the porosity of the metallic particle composition 120. If the particle composition 120 is disintegratable, the release rate or rate profile of the therapeutic agent is determined both by the porosity and the rate of disintegration of the metallic particle composition 120.

As shown in Fig. 3C, in some embodiments of the invention the metallic particulate composition 120 and the molecules of the therapeutic agent-containing composition 115 may be mixed together prior to compaction in the depressions 110d. In this way the therapeutic agent is released as the metallic particles disintegrate. This can provide a therapeutic agent release rate or rate profile that is different from the release rate or rate profile that is achieved when the metallic particle and drug molecules are segregated in the manner shown in Fig. 3A.

In yet other embodiments of the invention the metallic particulate composition 120 may comprise magnetic particles. For instance, the magnetic particles may be formed from a magnetic material such as a ferromagnetic metal or metal alloy, i.e., materials which exhibit good magnetic susceptibility. Examples of such materials include, without limitation, the magnetic metals iron (Fe), cobalt (Co), nickel (Ni), awaruite (Ni₃Fe) and wairauite (CoFe) and the magnetic oxides magnetite (Fe₃O₄), maghemite (Fe₂O₃) and magnesioferrite (MgFe₂O₄).

When the metallic particles are formed from a magnetic material, the substrate likewise can be formed from a magnetic material such as any of the aforementioned magnetic materials. Alternatively, the depressions in the substrate or the entire substrate itself can be coated with a magnetic material using, for instance, a deposition process such as physical vapor deposition, chemical vapor deposition, electrolysis, etc. In any case, the magnetic particles will be held in place within the depressions by virtue of the magnetic forces between the substrate and the magnetic particles. The magnetic particles act as a porous barrier layer to regulate the release of the therapeutic agent-containing composition. The magnetic particles may be capsules made of non-magnetic materials encapsulating a magnetic substance or particles made of a mixture of a nonmagnetic substance and a magnetic substance. In some cases the magnetic particles may be coated with a suitable material to reduce any undesirable effects that may be caused by the corrosive nature of the magnetic substance.

The magnetic particles can be used to further regulate the delivery rate of the therapeutic agent-containing composition by applying an external electromagnetic field to a patient in which the medical device is implanted. Generally, a suitable static magnetic field strength is within the range of 0.5 to 5 Tesla (Weber per square meter). The duration of the application may be determined based on various factors including the strength of the magnetic field, the magnetic material contained in the magnetic particles, the size of the particles and the desired release rate of the therapeutic agent-containing composition. The external magnetic field may be an oscillating electromagnetic field that causes vibration of the magnetic particles, which can enhance the release rate or initiate the release of a second therapeutic agent-containing composition. In addition, by increasing the frequency of the oscillating magnetic field, energy in the form of heat can be imparted to the magnetic particles. The elevation in temperature caused by the heat that is generated can be used to further influence the release rate of the therapeutic agent-containing composition. One skilled in the art can determine the proper cycle of the electromagnetic field, the proper intensity of the electromagnetic field, and the period of time over which the electromagnetic field is applied based on experiments and the like.

As shown in Fig. 3D, in some cases the depressions 110d can be laser welded or otherwise fused to provide a seal 125. The seal 125 can add further stability and delay the rate at which the metallic particulate composition disintegrates, thereby further regulating rate at which the therapeutic agent-containing composition is released. The seal 125 can be used in connection with any of the aforementioned embodiments of the invention.

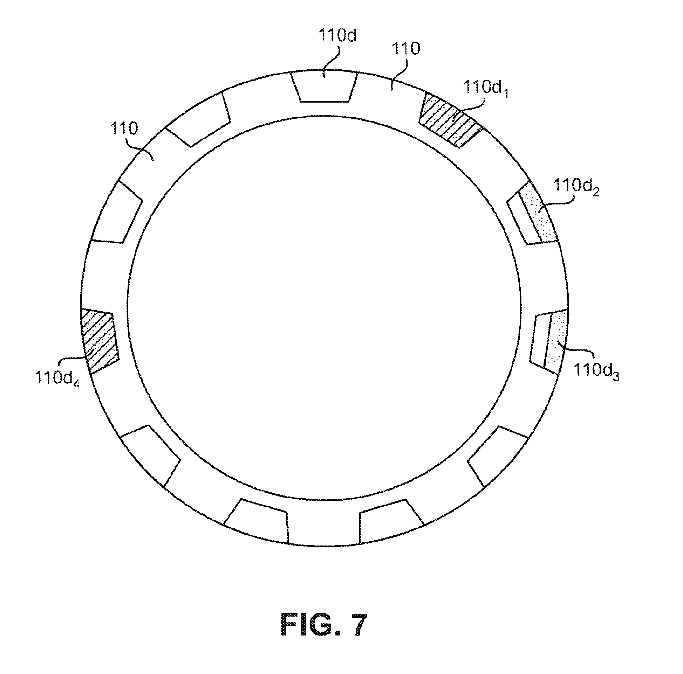

It should be noted that a drug releasing medical device constructed in accordance with the present invention may incorporate multiple depressions in which the depressions are all filled in the same way. For instance, all the depressions in the medical device may be filled as shown in any of Figs. 3A-3C. Alternatively, different depressions in the medical device may be filled differently. For example, in an embodiment like that of Fig. 7, some depressions such as depressions 110d₂ and 110d₃ may have the compacted metallic particles disposed over the therapeutic agent-containing composition, while other depressions such as depressions 110d₁ and 110d₄ may have the metallic particles mixed with the therapeutic-agent-containing composition, some of which may or may not be sealed with a seal 125.

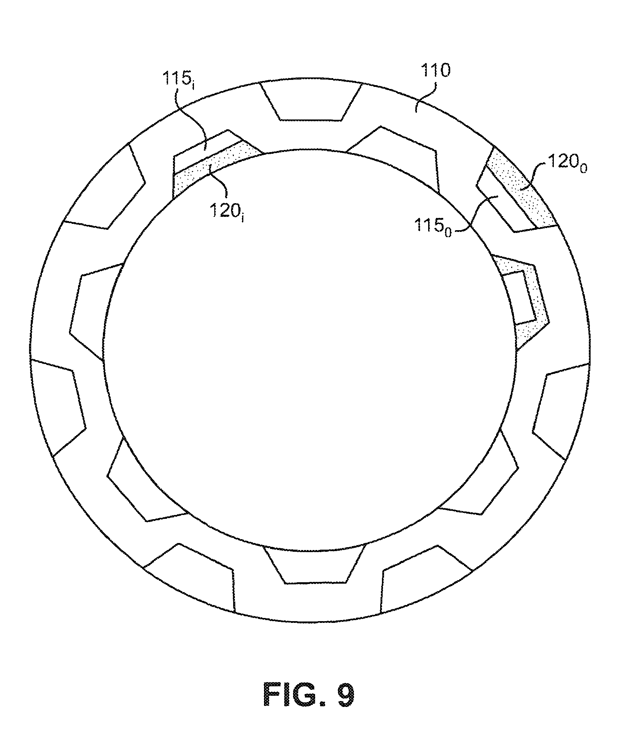

As indicated above, it is possible to provide different therapeutic agents at different locations on the substrate. In an embodiment like that of Fig. 9, for example, it is possible to provide one or more first depressions that are filled with a first therapeutic agent 115i (e.g., an anti-inflammatory agent, an endothelialization promoter or an antithrombotic agent) at the inner, luminal surface of the substrate 110, and one or more second depressions filled with a second therapeutic agent 115o that differs from the first therapeutic agent (e.g., an anti-restenotic agent) at the outer, abluminal surface of the substrate 110. Depressions that are filled with different therapeutic agents may be filled with the same or different metallic particulate compositions. Similarly, some of these depressions may have compacted metallic particles disposed over the therapeutic-agent-containing composition, some other depressions may have magnetic particles disposed over the therapeutic agent-containing composition, while still other depressions may have metallic particles mixed with the therapeutic agent-containing composition, some of which may or may not be sealed with a seal 125.

The substrate 110 may have single or multiple (e.g., 1 to 2 to 5 to 10 to 25 to 50 to 100 or more) therapeutic-agent-containing depressions. Therapeutic-agent-containing depression(s) may be provided over the entire device or only over one or more distinct portions of the device. For example, as seen from the above, for tubular devices such as stents, therapeutic-agent-filled depression(s) with associated compacted metallic particles may be provided on the luminal device surfaces, on the abluminal device surfaces, on the side surface, or a combination of two or more of the luminal, abluminal and side surfaces.

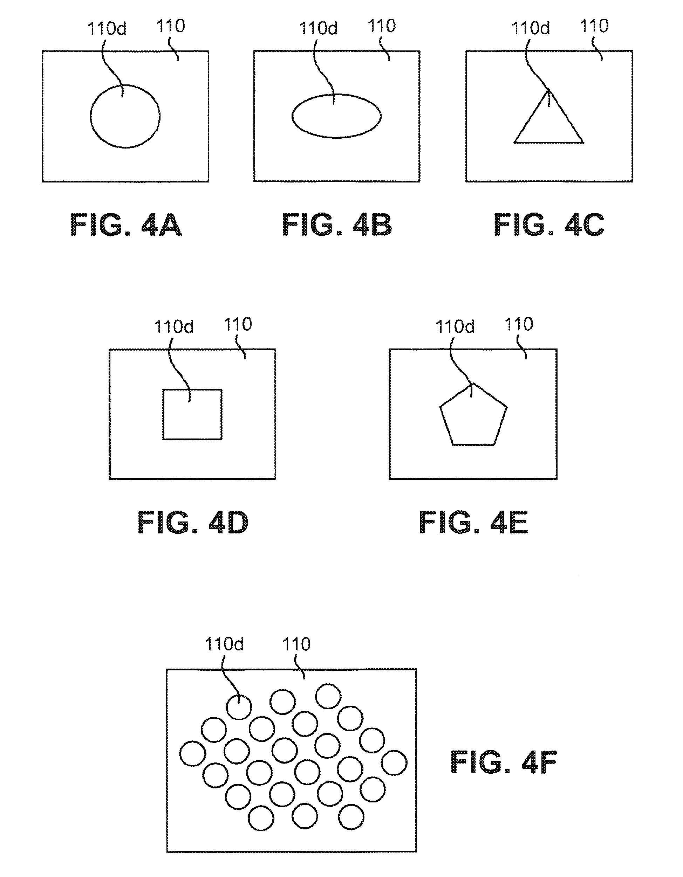

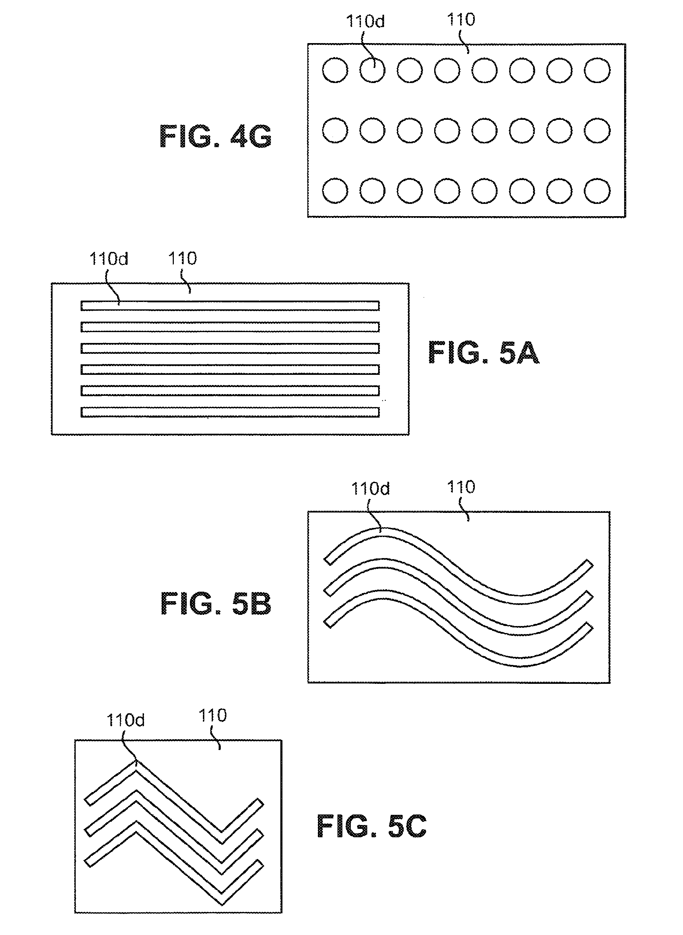

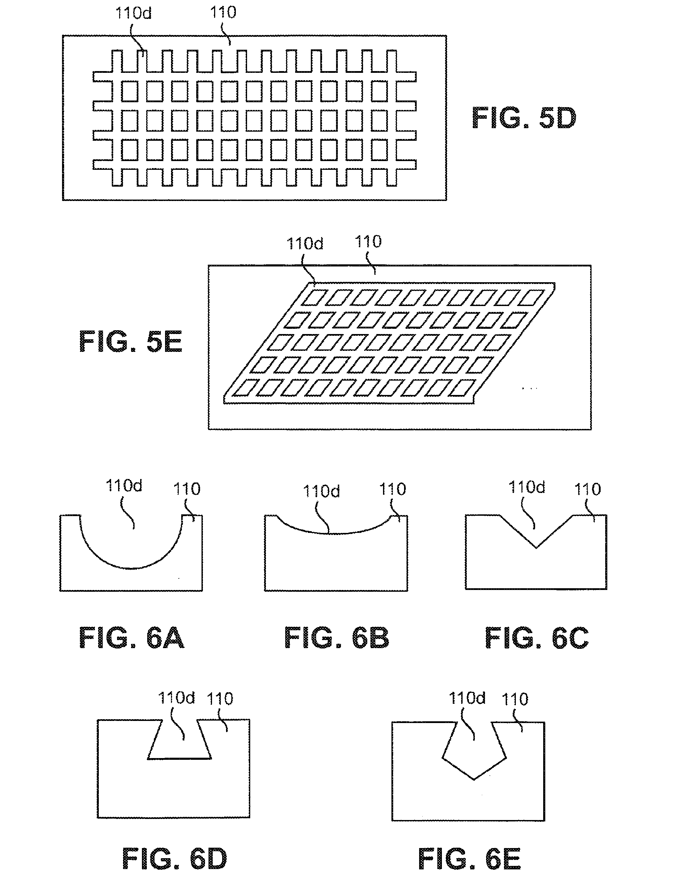

The depressions 110d which contain the therapeutic agents may come in various shapes and sizes. Examples include depressions whose lateral dimensions are circular (see, e.g., the top view of the circular hole of Fig. 4A, in which the depressed area 110d within the medical device substrate 110 is designated with a darker shade of grey), oval (see Fig. 4B), polygonal, for instance triangular (see Fig. 4C), quadrilateral (see Fig. 4D), penta-lateral (see Fig. 4E), as well as depressions of various other regular and irregular shapes and sizes. Multiple depressions 110d can be provided in a near infinite variety of arrays. See, e.g., the depressions 110d shown in Figs. 4F and 4G. Further examples of depressions 110d include trenches, such as simple linear trenches (see Fig. 5A), wavy trenches (see Fig. 5B), trenches formed from linear segments whose direction undergoes an angular change (see Fig. 5C), trench networks intersecting at right angles (see Fig. 5D), as well as other angles (see Fig. 5E), as well as other regular and irregular trench configurations.

The therapeutic agent-containing depressions can be of any size that provides the features of the invention. Commonly, the medical devices of the invention contain therapeutic agent-containing depressions whose smallest lateral dimension (e.g., the diameter for a cylindrical depression, the width for an elongated depression such a trench, etc.) is less than 10 mm (10000 µm), for example, ranging from 10,000 µm to 5000 µm to 2500 µm to 1000 µm to 500 µm to 250 µm to 100 µm to 50 µm to 10 µm to 5 µm to 2.5 µm to 1 µm or less.

As indicated above, the depressions 110d may be in the form of blind holes, trenches, etc. Such depressions 110d may have a variety of cross-sections, such as semicircular cross-sections (see, e.g., Fig. 6A), semi-oval cross-sections (see, e.g., Fig. 6B), polygonal cross-sections, including triangular (see, e.g., Fig. 6C), quadrilateral (see, e.g., Figs. 6D) and penta-lateral (see, e.g., Fig. 6E) cross-sections, as well as other regular and irregular cross-sections. In certain embodiments, the depressions are high aspect ratio depressions, meaning that the depth of the depression is greater than the width of the depression, for example, ranging from 1.5 to 2 to 2.5 to 5 to 10 to 25 or more times the width. In certain other embodiments, the depressions are low aspect ratio depressions, meaning that the depth of the depression is less than the width of the depression, for example, ranging from 0.75 to 0.5 to 0.4 to 0.2 to 0.1 to 0.04 or less times the width.

Examples of techniques for forming depressions in substrates (e.g., holes, trenches, etc.), include molding techniques, direct removal techniques, and mask-based removal techniques. In molding techniques, a mold may be provided with various protrusions, which after casting the substrate of interest, create depressions in the substrate. Various direct and mask-based removal techniques are discussed below.

As previously indicated, in the present invention, the depressions further contain (i.e., they are at least partially filled with) one or more therapeutic agents that may be used singly or in combination. The therapeutic agents may be present in pure form or admixed with another material, for example, a diluent, filler, excipient, matrix material, etc. Materials for these purposes may be selected, for example, from suitable members of the polymers listed below, among many other possible materials (e.g., small molecule chemical species). Where therapeutic agents are used in combination, one therapeutic agent may provide a matrix for another therapeutic agent.

By varying the size (i.e., volume) and number of the depressions, as well as the concentration of the therapeutic agents within the depressions, a range of therapeutic agent loading levels can be achieved. The amount of loading may be determined by those of ordinary skill in the art and may ultimately depend, for example, upon the disease or condition being treated, the age, sex and health of the subject, the nature (e.g., potency) of the therapeutic agent, or other factors.

The substrate material in which the depressions are formed may vary widely in composition and is not limited to any particular material. When magnetic particles are employed, magnetic materials such as those mentioned above should be used. When non-magnetic metallic particles are employed, the substrate material can be selected from a range of biostable materials and biodisintegrable materials, including (a) organic materials (i.e., materials containing organic species, typically 50 wt% or more, for example, from 50 wt% to 75 wt% to 90 wt% to 95 wt% to 97.5 wt% to 99 wt% or more) such as polymeric materials and biologics, (b) inorganic materials (i.e., materials containing inorganic species, typically 50 wt% or more, for example, from 50 wt% to 75 wt% to 90 wt% to 95 wt% to 97.5 wt% to 99 wt% or more), such as metallic materials (i.e., materials containing metals, typically 50 wt% or more, for example, from 50 wt% to 75 wt% to 90 wt% to 95 wt% to 97.5 wt% to 99 wt% or more) and non-metallic inorganic materials (i.e., materials containing non-metallic inorganic materials, typically 50 wt% or more, for example, from 50 wt% to 75 wt% to 90 wt% to 95 wt% to 97.5 wt% to 99 wt% or more) (e.g., carbon, semiconductors, glasses and ceramics, which may contain various metal- and non-metal-oxides, various metal- and non-metal-nitrides, various metal- and non-metal-carbides, various metal- and non-metal-borides, various metal- and non-metal-phosphates, and various metal- and non-metal-sulfides, among others), and (c) hybrid materials (e.g., hybrid organic-inorganic materials, for instance, polymer/metallic inorganic and polymer/non-metallic inorganic hybrids).

Specific examples of non-metallic inorganic materials may be selected, for example, from materials containing one or more of the following: metal oxides, including aluminum oxides and transition metal oxides (e.g., oxides of titanium, zirconium, hafnium, tantalum, molybdenum, tungsten, rhenium, iron, niobium, and iridium); silicon; silicon-based ceramics, such as those containing silicon nitrides, silicon carbides and silicon oxides (sometimes referred to as glass ceramics); calcium phosphate ceramics (e.g., hydroxyapatite); carbon; and carbon-based, ceramic-like materials such as carbon nitrides.

Specific examples of metallic inorganic materials may be selected, for example, from metals (e.g., metals such as gold, niobium, platinum, palladium, iridium, osmium, rhodium, titanium, tantalum, tungsten, ruthenium, iron, zinc and magnesium), metal alloys comprising iron and chromium (e.g., stainless steels, including platinum-enriched radiopaque stainless steel), alloys comprising nickel and titanium (e.g., Nitinol), alloys comprising cobalt and chromium, including alloys that comprise cobalt, chromium and iron (e.g., elgiloy alloys), alloys comprising nickel, cobalt and chromium (e.g., MP 35N), alloys comprising cobalt, chromium, tungsten and nickel (e.g., L605), alloys comprising nickel and chromium (e.g., inconel alloys), and biodegradable alloys of magnesium, zinc and/or iron.

As previously noted, in some embodiments of the invention, the therapeutic-agent releasing medical device is preferably polymer-free. However, in other embodiments the substrate from which medical device is fabricated may be formed from polymers (biostable or biodegradable) as well as other high molecular weight organic materials, and may be selected, for example, from suitable materials containing one or more of the following: polycarboxylic acid polymers and copolymers including polyacrylic acids; acetal polymers and copolymers; acrylate and methacrylate polymers and copolymers (e.g., n-butyl methacrylate); cellulosic polymers and copolymers, including cellulose acetates, cellulose nitrates, cellulose propionates, cellulose acetate butyrates, cellophanes, rayons, rayon triacetates, and cellulose ethers such as carboxymethyl celluloses and hydroxyalkyl celluloses; polyoxymethylene polymers and copolymers; polyimide polymers and copolymers such as polyether block imides, polyamidimides, polyesterimides, and polyetherimides; polysulfone polymers and copolymers including polyarylsulfones and polyethersulfones; polyamide polymers and copolymers including nylon 6,6, nylon 12, polyether-block co-polyamide polymers (e.g., Pebax® resins), polycaprolactams and polyacrylamides; resins including alkyd resins, phenolic resins, urea resins, melamine resins, epoxy resins, allyl resins and epoxide resins; polycarbonates; polyacrylonitriles; polyvinylpyrrolidones (cross-linked and otherwise); polymers and copolymers of vinyl monomers including polyvinyl alcohols, polyvinyl halides such as polyvinyl chlorides, ethylene-vinylacetate copolymers (EVA), polyvinylidene chlorides, polyvinyl ethers such as polyvinyl methyl ethers, vinyl aromatic polymers and copolymers such as polystyrenes, styrene-maleic anhydride copolymers, vinyl aromatic-hydrocarbon copolymers including styrene-butadiene copolymers, styrene-ethylene-butylene copolymers (e.g., a polystyrene-polyethylene/butylene-polystyrene (SEBS) copolymer, available as Kraton® G series polymers), styreneisoprene copolymers (e.g., polystyrene-polyisoprene-polystyrene), acrylonitrile-styrene copolymers, acrylonitrile-butadiene-styrene copolymers, styrene-butadiene copolymers and styrene-isobutylene copolymers (e.g., polyisobutylene-polystyrene block copolymers such as SIBS), polyvinyl ketones, polyvinylcarbazoles, and polyvinyl esters such as polyvinyl acetates; polybenzimidazoles; ionomers; polyalkyl oxide polymers and copolymers including polyethylene oxides (PEO); polyesters including polyethylene terephthalates, polybutylene terephthalates and aliphatic polyesters such as polymers and copolymers of lactide (which includes lactic acid as well as d-,1- and meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (a copolymer of polylactic acid and polycaprolactone is one specific example); polyether polymers and copolymers including polyarylethers such as polyphenylene ethers, polyether ketones, polyether ether ketones; polyphenylene sulfides; polyisocyanates; polyolefin polymers and copolymers, including polyalkylenes such as polypropylenes, polyethylenes (low and high density, low and high molecular weight), polybutylenes (such as polybut-1-ene and polyisobutylene), polyolefin elastomers (e.g., santoprene), ethylene propylene diene monomer (EPDM) rubbers, poly-4-methyl-pen-1-enes, ethylene-alpha-olefin copolymers, ethylene-methyl methacrylate copolymers and ethylene-vinyl acetate copolymers; fluorinated polymers and copolymers, including polytetrafluoroethylenes (PTFE), poly(tetrafluoroethylene-co-hexafluoropropene) (FEP), modified ethylene-tetrafluoroethylene copolymers (ETFE), and polyvinylidene fluorides (PVDF); silicone polymers and copolymers; polyurethanes; p-xylylene polymers; polyiminocarbonates; copoly(ether-esters) such as polyethylene oxide-polylactic acid copolymers; polyphosphazines; polyalkylene oxalates; polyoxaamides and polyoxaesters (including those containing amines and/or amido groups); polyorthoesters; biopolymers, such as polypeptides, proteins, polysaccharides and fatty acids (and esters thereof), including fibrin, fibrinogen, collagen, elastin, chitosan, gelatin, starch, glycosaminoglycans such as hyaluronic acid; as well as blends and further copolymers of the above.

As previously noted, a variety of different techniques may be employed to form the depressions or to sculpt a medical device from the substrate (e.g., to sculpt stent struts from tubes). For example, such techniques include direct removal techniques as well as mask-based removal techniques, in which masking is used to protect material that is not to be removed. Direct removal techniques include those in which material is removed through contact with solid tools (e.g., microdrilling, micromachining, etc., using high precision equipment such as high precision milling machines and lathes) and those that remove material without the need for solid tools (e.g., those based on directed energetic beams such as laser, electron, and ion beams). In the latter cases, techniques based on diffractive optical elements (DOEs), holographic diffraction, and/or polarization trepanning, among other beam manipulation methods, may be employed to generate patterns as desired. Using these and other techniques, multiple depressions can be formed in a material layer at once.

Mask-based techniques include those in which the masking material contacts the material to be machined (e.g., where masks are formed using known lithographic techniques, including optical, ultraviolet, deep ultraviolet, electron beam, and x-ray lithography) and techniques in which the masking material does not contact the material to be machined, but which is provided between a directed source of excavating energy and the material to be machined (e.g., opaque masks having apertures formed therein, as well as semi-transparent masks such as gray-scale masks which provide variable beam intensity and thus variable machining rates). One process, known as columnated plasma lithography, is capable of producing X-rays for lithography having wavelengths on the order of 10 nm. Material is removed in regions not protected by the above masks using any of a range of processes including physical processes (e.g., thermal sublimation and/or vaporization of the material that is removed), chemical processes (e.g., chemical breakdown and/or reaction of the material that is removed), or a combination of both. Specific examples of removal processes include wet and dry (plasma) etching techniques, and ablation techniques based on directed energetic beams such as electron, ion and laser beams. A lithography-based process for forming nanoporous silicon is described, for example, in L. Leoni et al. "Nanoporous Platforms for Cellular Sensing and Delivery," *Sensors* 2002, *2*, 111-120.

In those embodiments of the invention where laser light is used for material removal (e.g., for formation of depressions, stent struts, etc.), shorter wavelength light may be preferred. There are several reasons for this. For example, shorter wavelength light such as UV and deep-UV light can be imaged to a smaller spot size than light of longer wavelengths (e.g., because the minimum feature size is limited by diffraction, which increases with wavelength). Such shorter wavelength light is also typically relatively photolytic, displaying less thermal influence on surrounding material. Moreover, many materials have high absorption coefficients in the ultraviolet region. This means that the penetration depth is small, with each pulse removing only a thin layer of material, thereby allowing precise control of the drilling depth. Various lasers are available for laser ablation, including excimer lasers, solid state lasers such as those based on Nd:YAG and Nd:vanadate, among other crystals, metal vapor lasers, such as copper vapor lasers, and femtosecond lasers. Further information on lasers and laser ablation may be found in T. Lippert et al., "Chemical and spectroscopic aspects of polymer ablation: Special features and novel directions," Chem. Rev., 103(2): 453-485 Feb. 2003; J. Meijer et al., "Laser Machining by short and ultrashort pulses, state of the art and new opportunities in the age of photons," Annals of the CIRP, 51(2), 531-550, 2002, and U.S. Patent No. 6,517,888 to Weber.

It is noted that there is a great amount of available know-how in the semiconductor industry for etching holes (e.g., vias), trenches and other voids in various materials. For this reason, in some embodiments of the invention, material may be removed from materials for which processing is routine in the semiconducting industry including semiconducting materials such as silicon, conductive materials such as metals and metal alloys, and insulating materials such as silicon oxide, silicon nitride and various metal oxides.

"Biologically active agents," "drugs," "therapeutic agents," "pharmaceutically active agents," "pharmaceutically active materials," and other related terms may be used interchangeably herein and include genetic therapeutic agents, non-genetic therapeutic agents and cells. A wide variety of therapeutic agents can be employed in conjunction with the present invention. Numerous therapeutic agents are described here.

Suitable non-genetic therapeutic agents for use in connection with the present invention may be selected, for example, from one or more of the following:: (a) anti-thrombotic agents such as heparin, heparin derivatives, urokinase, clopidogrel, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/ antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) smooth muscle relaxants such as alpha receptor antagonists (e.g., doxazosin, tamsulosin, terazosin, prazosin and alfuzosin), calcium channel blockers (e.g., verapimil, diltiazem, nifedipine, nicardipine, nimodipine and bepridil), beta receptor agonists (e.g., dobutamine and salmeterol), beta receptor antagonists (e.g., atenolol, metaprolol and butoxamine), angiotensin-II receptor antagonists (e.g., losartan, valsartan, irbesartan, candesartan, eprosartan and telmisartan), and antispasmodic/anticholinergic drugs (e.g., oxybutynin chloride, flavoxate, tolterodine, hyoscyamine sulfate, diclomine), (u) bARKct inhibitors, (v) phospholamban inhibitors, (w) Serca 2 gene/protein, (x) immune response modifiers including aminoquizolines, for instance, imidazoquinolines such as resiquimod and imiquimod, (y) human apolioproteins (e.g., AI, AII, AIII, AIV, AV, etc.), (z) selective estrogen receptor modulators (SERMs) such as raloxifene, lasofoxifene, arzoxifene, miproxifene, ospemifene, PKS 3741, MF 101 and SR 16234, (aa) PPAR agonists such as rosiglitazone, pioglitazone, netoglitazone, fenofibrate, bexaotene, metaglidasen, rivoglitazone and tesaglitazar, (bb) prostaglandin E agonists such as alprostadil or ONO 8815Ly, (cc) thrombin receptor activating peptide (TRAP), (dd) vasopeptidase inhibitors including benazepril, fosinopril, lisinopril, quinapril, ramipril, imidapril, delapril, moexipril and spirapril, (ee) thymosin beta 4, and (ff) phospholipids including phosphorylcholine, phosphatidylinositol and phosphatidylcholine.

Preferred non-genetic therapeutic agents include taxanes such as paclitaxel (including particulate forms thereof, for instance, protein-bound paclitaxel particles such as albumin-bound paclitaxel nanoparticles, e.g., ABRAXANE), sirolimus, everolimus, tacrolimus, zotarolimus, Epo D, dexamethasone, estradiol, halofuginone, cilostazole, geldanamycin, ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, Serca 2 gene/protein, imiquimod, human apolioproteins (e.g., AI-AV), growth factors (e.g., VEGF-2), as well derivatives of the forgoing, among others.

Suitable genetic therapeutic agents for use in connection with the present invention include anti-sense DNA and RNA as well as DNA coding for the various proteins (as well as the proteins themselves) and may be selected, for example, from one or more of the following:: (a) anti-sense RNA, (b) tRNA or rRNA to replace defective or deficient endogenous molecules, (c) angiogenic and other factors including growth factors such as acidic and basic fibroblast growth factors, vascular endothelial growth factor, endothelial mitogenic growth factors, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers such as polyvinylpyrrolidone (PVP), SP1017 (SUPRATEK), lipids such as cationic lipids, liposomes, lipoplexes, nanoparticles, or microparticles, with and without targeting sequences such as the protein transduction domain (PTD).

Cells for use in conjunction with the present invention include cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

Further therapeutic agents, not necessarily exclusive of those listed above, have been identified as candidates for vascular treatment regimens, for example, as agents targeting restenosis (anti-restenotic agents). Suitable agents may be selected, for example, from one or more of the following:: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists such as bosentan, sitaxsentan sodium, atrasentan, endonentan, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) Angiotensin Converting Enzyme (ACE) inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, atorvastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD (orgotein), SOD mimics, verteporfin, rostaporfin, AGI 1067, and M 40419, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) matrix metalloprotease (MMP) pathway inhibitors such as marimastat, ilomastat, metastat, batimastat, pentosan polysulfate, rebimastat, incyclinide, apratastat, PG 116800, RO 1130830 or ABT 518, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate , nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), rapamycin (sirolimus) and its analogs (e.g., everolimus, tacrolimus, zotarolimus, etc.), cerivastatin, flavopiridol and suramin, (aa) matrix deposition/organization pathway inhibitors such as halofuginone or other quinazolinone derivatives, pirfenidone and tranilast, (bb) endothelialization facilitators such as VEGF and RGD peptide, (cc) blood rheology modulators such as pentoxifylline and (dd) glucose cross-link breakers such as alagebrium chloride (ALT-711).

Numerous additional therapeutic for the practice of the present invention may be selected from suitable therapeutic agents disclosed in U.S. Patent No. 5,733,925 to Kunz.

## Claims

1. An implantable or insertable medical device comprising as components:
a substrate component (110) comprising one ore more depressions (110d) which are at least partially filled with a therapeutic agent-containing material (115) that comprises a therapeutic agent,
**characterized by**
a metallic particulate composition (120) disposed in the depression such that it covers the therapeutic agent-containing material (115) and regulates transport of the therapeutic agent between the depression and the exterior of the device upon implantation or insertion of the device into a subject.

2. The implantable or insertable medical device of claim 1 wherein the particulate composition and the substrate are magnetic such that the particulate composition is retained in the cavity by magnetic force.

3. The implantable or insertable medical device of claim 1 or 2 wherein the particulate composition comprises compacted particles.

4. The implantable or insertable medical device of one of the preceeding claims wherein the metallic particles are biodisintegratable.

5. The implantable or insertable medical device of one of the preceeding claims wherein the particulate composition includes porous channels through which the therapeutic agent can be released.

6. The implantable or insertable medical device of one of the preceeding claims further comprising a seal disposed over the particulate composition in the depression to delay release of the therapeutic agent.

7. The implantable or insertable medical device of one of the preceeding claims, wherein said depressions are blind holes or trenches.

8. The implantable or insertable medical device of one of the preceeding claims, wherein said medical device is adapted for implantation or insertion into the coronary vasculature, peripheral vascular system, esophagus, trachea, colon, biliary tract, urogenital system, or brain.

9. The implantable or insertable medical device of one of the preceeding claims, wherein said medical device is selected from a drug delivery device, an implant, a stent, a graft, a filter, a catheter, a defibrillator, a chronic rhythm management lead and a neuromodulation device.

10. The implantable or insertable medical device of one of the preceeding claims, wherein the therapeutic-agent-containing material further comprises a material in addition to said therapeutic agent.

11. The implantable or insertable medical device of one of the preceeding claims, wherein the therapeutic-agent-containing material further comprises another therapeutic agent.

12. The implantable or insertable medical device of one of the preceeding claims, wherein said therapeutic agent is selected from one or more of the group consisting of anti-thrombotic agents, antiproliferative agents, anti-inflammatory agents, anti-restenotic agents, anti-migratory agents, agents affecting extracellular matrix production and organization, antineoplastic agents, anti-mitotic agents, anesthetic agents, anti-coagulants, vascular cell growth promoters, vascular cell growth inhibitors, cholesterol-lowering agents, vasodilating agents, TGF-[beta] elevating agents, and agents that interfere with endogenous vasoactive mechanisms.

13. A method of making the implantable or insertable medical device of one of the preceeding claims, comprising
(a) at least partially filling the depressions (110d) with the therapeutic agent-containing material (115), and
(b) applying the metallic particulate composition (120) over the therapeutic agent-containing material.

14. The method of claim 13 wherein the particulate composition and the substrate are magnetic such that the particulate composition is retained in the cavity by magnetic force.

15. The method of claim 13 or 14 further comprising compacting the particulate composition in the depression.

## Patentansprüche

1. Ein implantierbares oder einsetzbares medizinisches Gerät umfassend als Komponenten:
eine Substratkomponente (110) umfassend eine oder mehrere Einsenkungen (110d), die zumindest teilweise gefüllt sind mit einem thereapeutischen Wirkstoff beinhaltenden Material (115) das einen therapeutischen Wirkstoff umfasst,
**gekennzeichnet durch**
eine metallische partikelförmige Mischung (120) angebracht in den Einsenkungen so, dass sie das therapeutische Wirkstoff beinhaltende Material (115) bedeckt und Transport des therapeutischen Wirkstoffs zwischen den Einsenkungen und der Außenseite des Gerätes nach Implantation oder Einsetzen des Gerätes in ein Subjekt reguliert.

2. Das implantierbare oder einsetzbare medizinische Gerät nach Anspruch 1, wobei die partikelförmige Mischung und das Substrat derart magnetisch sind, dass die partikelförmige Mischung in dem Hohlraum durch magnetische Kräfte gehalten wird.

3. Das implantierbare oder einsetzbare medizinische Gerät nach Anspruch 1 oder 2, wobei die partikelförmige Mischung verdichtete Partikel umfasst.

4. Das implantierbare oder einsetzbare medizinische Gerät nach einem der vorhergehenden Ansprüche, wobei die metallischen Partikel biodesintegrierbar sind.

5. Das implantierbare oder einsetzbare medizinische Gerät nach einem der vorhergehenden Ansprüche, wobei die partikelförmige Mischung poröse Kanäle beinhaltet durch die der therapeutische Wirkstoff freigesetzt werden kann.

6. Das implantierbare oder einsetzbare medizinische Gerät nach einem der vorhergehenden Ansprüche weiter umfassend eine Abdichtung angebracht über der partikelförmigen Mischung in der Einsenkung, um die Freisetzung des therapeutischen Wirkstoffs zu verzögern.

7. Das implantierbare oder einsetzbare medizinische Gerät nach einem der vorhergehenden Ansprüche wobei die Einsenkungen Sacklöcher oder Einschnitte sind.

8. Das implantierbare oder einsetzbare medizinische Gerät nach einem der vorhergehenden Ansprüche, wobei das medizinische Gerät ausgelegt ist zur Implantation oder zum Einsetzen in die Koronargefäße, das periphere vaskuläre System, den Ösophagus, die Luftröhre, den Dickdarm, die Gallenwege, das Urogenitalsystem, oder das Gehirn.

9. Das implantierbare oder einsetzbare medizinische Gerät nach einem der vorhergehenden Ansprüche, wobei das medizinische Gerät ausgewählt ist aus einem Wirkstoffzuführgerät, einem Implantat, einem Stent, einem Graft, einem Filter, einem Katheter, einem Defibrillator, einer chronischen Schrittmachersonde und ein Neuromodulationsgerät.

10. Das implantierbare oder einsetzbare medizinische Gerät nach einem der vorhergehenden Ansprüche, wobei das therapeutische Wirkstoff beinhaltende Material weiter umfasst ein zusätzliches Material zu dem therapeutischen Wirkstoff.

11. Das implantierbare oder einsetzbare medizinische Gerät nach einem der vorhergehenden Ansprüche, wobei das therapeutische Wirkstoff beinhaltende Material weiter umfasst einen anderen therapeutischen Wirkstoff.

12. Das implantierbare oder einsetzbare medizinische Gerät nach einem der vorhergehenden Ansprüche, wobei der therapeutische Wirkstoff ausgewählt ist aus einer oder mehreren der Gruppen bestehend aus antithrombotische Wirkstoffe, anti-proliferative Wirkstoffe, entzündungshemmende Wirkstoffe, anti-restenotische Wirkstoffe, Antimigrationswirkstoffe, Wirkstoffe die die extrazelluläre Matrixproduktion und -organisation betreffen, anti-neoplastische Wirkstoffe, anti-mitotische Wirkstoffe, anästhetische Wirkstoffe, Antikoagulantien, vaskuläre Zellwachstumspromotoren, vaskuläre Zellwachstumsinhibitoren, Cholesterol senkende Wirkstoffe, vasodilatierende Wirkstoffe, TGF-[beta] erhöhende Wirkstoffe und Wirkstoffe, die mit endogenen vasoaktiven Mechanismen interferieren.

13. Eine Methode zum Herstellen des implantierbaren oder einsetzbaren medizinischen Geräts nach einem der vorhergehenden Ansprüche umfassend
(a) zumindest teilweises Füllen der Einsenkungen (110d) mit dem therapeutischen Wirkstoff beinhaltenden Material
(b) anbringen der metallischen partikelförmigen Mischung (120) über das therapeutische Wirkstoff beinhaltende Material.

14. Das Verfahren nach Anspruch 13, wobei die partikelförmige Mischung und das Substrat derart magnetisch sind, dass die partikelförmige Mischung in dem Hohlraum durch magnetische Kräfte gehalten wird.

15. Das Verfahren nach Anspruch 13 oder 14 weiter umfassend verdichten der partikelförmigen Mischung in die Einsenkung.

## Revendications

1. Dispositif médical implantable ou insérable comprenant en tant que composants : un composant de substrat (110) comprenant une ou plusieurs dépressions (110d) qui sont au moins partiellement remplies avec un matériau contenant un agent thérapeutique (115) qui comprend un agent thérapeutique,
**caractérisé par**
une composition particulaire métallique (120) disposée dans la dépression de sorte qu'elle couvre le matériau contenant un agent thérapeutique (115) et régule le transport de l'agent thérapeutique entre la dépression et l'extérieur du dispositif après implantation ou insertion du dispositif dans un sujet.

2. Dispositif médical implantable ou insérable de la revendication 1 dans lequel la composition particulaire et le substrat sont magnétiques de sorte que la composition particulaire soit retenue dans la cavité par une force magnétique.

3. Dispositif médical implantable ou insérable de la revendication 1 ou 2 dans lequel la composition particulaire comprend des particules compactées.

4. Dispositif médical implantable ou insérable d'une des revendications précédentes dans lequel les particules métalliques sont biodésintégrables.

5. Dispositif médical implantable ou insérable d'une des revendications précédentes dans lequel la composition particulaire comprend des canaux poreux à travers lesquels l'agent thérapeutique peut être libéré.

6. Dispositif médical implantable ou insérable d'une des revendications précédentes comprenant en outre un scellement disposé sur la composition particulaire dans la dépression pour retarder la libération de l'agent thérapeutique.

7. Dispositif médical implantable ou insérable d'une des revendications précédentes, dans lequel lesdites dépressions sont des trous borgnes ou des tranchées.

8. Dispositif médical implantable ou insérable d'une des revendications précédentes, dans lequel ledit dispositif médical est adapté pour implantation ou insertion dans la vascularisation coronaire, le système vasculaire périphérique, l'oesophage, la trachée, le côlon, les voies biliaires, le système urogénital, ou le cerveau.

9. Dispositif médical implantable ou insérable d'une des revendications précédentes, dans lequel ledit dispositif médical est choisi parmi un dispositif d'administration de médicament, un implant, une endoprothèse, une greffe, un filtre, un cathéter, un défibrillateur, une dérivation de contrôle du rythme chronique et un dispositif de neuromodulation.

10. Dispositif médical implantable ou insérable d'une des revendications précédentes, dans lequel le matériau contenant un agent thérapeutique comprend en outre un matériau en plus dudit agent thérapeutique.

11. Dispositif médical implantable ou insérable d'une des revendications précédentes, dans lequel le matériau contenant un agent thérapeutique comprend en outre un autre agent thérapeutique.

12. Dispositif médical implantable ou insérable d'une des revendications précédentes, dans lequel ledit agent thérapeutique est choisi parmi l'un ou plusieurs du groupe constitué d'agents antithrombotiques, d'agents antiprolifératifs, d'agents anti-inflammatoires, d'agents antiresténose, d'agents antimigratoires, d'agents affectant la production et l'organisation de matrice extracellulaire, d'agents antinéoplasiques, d'agents antimitotiques, d'agents anesthésiques, d'anticoagulants, de promoteurs de croissance des cellules vasculaires, d'inhibiteurs de croissance des cellules vasculaires, d'agents hypocholestérolémiants, d'agents vasodilatateurs, d'agents d'élévation de TGF-β, et d'agents qui interfèrent avec des mécanismes endogènes vasoactifs.

13. Procédé de fabrication du dispositif médical implantable ou insérable d'une des revendications précédentes, comprenant
(a) remplir au moins partiellement les dépressions (110d) avec le matériau contenant un agent thérapeutique (115), et
(b) l'application de la composition particulaire métallique (120) sur le matériau contenant un agent thérapeutique.

14. Procédé de la revendication 13 dans lequel la composition particulaire et le substrat sont magnétiques de sorte que la composition particulaire soit retenue dans la cavité par une force magnétique.

15. Procédé de la revendication 13 ou 14 comprenant en outre le compactage de la composition particulaire dans la dépression.
